(19) Europäisches Patentamt
European Patent Office
Office européen des brevets

(11) Veröffentlichungsnummer: **0 034 240**
**B1**

(12) # EUROPÄISCHE PATENTSCHRIFT

(45) Veröffentlichungstag der Patentschrift: **13.04.83**

(51) Int. Cl.³: **C 07 C 102/08,** C 07 C 103/30 // C07D253/06

(21) Anmeldenummer: **81100034.8**

(22) Anmeldetag: **07.01.81**

(54) **Verfahren zur Herstellung von alpha-Ketocarbonsäure-N-tert.-butylamiden.**

(30) Priorität: **22.01.80 DE 3002203**

(43) Veröffentlichungstag der Anmeldung: **26.08.81 Patentblatt 81/34**

(45) Bekanntmachung des Hinweises auf die Patenterteilung: **13.04.83 Patentblatt 83/15**

(84) Benannte Vertragsstaaten: **BE CH DE FR GB IT LI NL**

(56) Entgegenhaltungen:
**DE-A-2 733 180**
**DE-A-2 733 181**
**US-A-2 518 156**

(73) Patentinhaber: **BAYER AG, Zentralbereich Patente, Marken und Lizenzen, D-5090 Leverkusen 1, Bayerwerk (DE)**

(72) Erfinder: **Bonse, Gerhard, Dr., Wolfskaul 3, D-5000 Köln 80 (DE)**
Erfinder: **Blank, Heinz Ulrich, Dr., Am Geusfelde 35, D-5068 Odenthal (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 034 240 B1

Verfahren zur Herstellung von α-Ketocarbonsäure-N-tert.-butylamiden

Die vorliegende Erfindung betrifft ein neues Verfahren zur Herstellung von α-Ketocarbonsäure-N-tert.-butylamiden, welche als Zwischenprodukte zur Synthese von bekannten herbiziden Wirkstoffen verwendet werden können.

Allgemein sind α-Ketocarbonsäureamide wertvolle Zwischenprodukte zur Herstellung von herbizid wirksamen 1,2,4-Triazin-5-on-Derivaten, die z. B. gemäß DE-OS 2 165 554 aus α-Ketocarbonsäureamiden und Hydrazinderivaten gut zugänglich sind.

Es ist bereits bekanntgeworden, sekundäre und tertiäre Alkohole, Olefine oder Ester mit Nitrilen in Gegenwart von Säuren, beispielsweise Schwefelsäure oder auch Lewis-Säuren, zu N-alkylsubstituierten Carbonsäureamiden umzusetzen (vgl. z. B. J. J. Ritter et al., J. Amer. Chem. Soc. 70, S. 4045 [1948]).

Diese Reaktion (in der Literatur als »Ritter-Reaktion« bekannt) kann mit zahlreichen aliphatischen und aromatischen Mono- und Dinitrilen, mit ungesättigten Nitrilen und Aldehydcyanhydrinen durchgeführt werden (vgl. z. B. J. Amer. Chem. Soc. 71, S. 4128 [1949]; 71, S. 4130 [1949]; 72, S. 5577 [1950]; 73, S. 4076 [1951]; ferner »Organic Reactions«, John Wiley & Sons, Inc. New York [1969], Vol. 17, S. 213–335; »Methodicum Chimicum«, Georg Thieme Verlag Stuttgart [1974], Band 6, S. 710; »Reaktionen der Organischen Chemie« von H. Krauch und W. Kunz, Dr. Alfred Hüthig Verlag Heidelberg [1976], S. 544).

Es ist weiterhin bekannt, daß in gleicher Weise auch Acylcyanide der Ritter-Reaktion zugänglich sind. So wird z. B. in Acta Chem. Scand. 22, S. 1787–1790 (1968) die Herstellung von Phenylglyoxylsäure-N-tert.-butylamid aus Benzoylcyanid und tert.-Butanol in Gegenwart von Lewis-Säuren in einer Ausbeute von 72% der Theorie beschrieben.

Nach DE-OS 2 733 180 und DE-OS 2 733 181 gelingt weiterhin die Darstellung von Phenylglyoxylsäure-N-tert.-butylamid und anderen Glyoxylsäure-N-tert.-butylamiden durch Umsetzung der entsprechenden Acylcyanide mit tert.-Butanol bzw. Isobutylen als tert.-Butylcarboniumionen bildende Komponenten in Gegenwart von starken Säuren (z. B. konzentrierter Schwefelsäure), d. h. unter den Bedingungen der Ritter-Reaktion, in Ausbeuten von 54–95% der Theorie.

Alternative Verfahren zur Herstellung von α-Ketocarbonsäuren, wie z. B. die Addition von Säurechloriden an Isonitrile (vgl. z. B. Chem. Ber. 94, S. 1116–1121 [1961]) oder die Oxidation von entsprechenden α-Hydroxycarbonsäureamiden mittels Schwermetalloxiden (vgl. z. B. DE-OS 2 208 568) sind — bedingt durch die Giftigkeit und den üblen Geruch der Isonitrile bzw. durch die Giftigkeit und die hohen Kosten der Schwermetalloxide — technisch wesentlich aufwendiger.

Es ist jedoch wünschenswert, die Ritter-Reaktion so zu verbessern, daß sich die benötigten α-Ketocarbonsäureamide durch Umsetzung von leicht zugänglichen Acylcyaniden (vgl. z. B. Angew. Chem. 68, S. 425–435 [1965]) mit einer geeigneten Carboniumionen bildenden Komponente in technisch einfacher Weise erhalten lassen.

Wie aus dem Schrifttum bekannt ist, wird der tert.-Butylmethyläther in Gegenwart von Schwefelsäure unter Bildung von Isobutylen zersetzt (vgl. J. Amer. Chem. Soc. 54, S. 2093 [1932]), so daß seine Nutzung als tert.-Butylcarboniumionen-Quelle unter den Bedingungen der Ritter-Reaktion erwartet werden konnte. Dementsprechend gelingt es gemäß US-PS 2 518 156, Benzoesäure-N-tert.-butylamid durch Umsetzung von Benzonitril mit tert.-Butylmethyläther herzustellen (Ausbeute 85% der Theorie).

Nach der Literaturstelle J. Amer. Chem. Soc. 70, S. 4045 (1948) kann das Benzoesäure-N-tert.-butylamid aus Benzonitril und Isobutylen sogar in einer Ausbeute von 90% der Theorie hergestellt werden.

Technische Vorteile bei der Verwendung von tert.-Butylmethyläther gegenüber Isobutylen oder tert.-Butanol als tert.-Butylcarboniumionen-Quelle in der Ritter-Reaktion sind bisher dem Stand der Technik nicht zu entnehmen.

Nach DE-OS 2 733 181 werden zur Herstellung von N-alkylsubstituierten α-Ketocarbonsäureamiden die Reaktionspartner in solchen Mengen eingesetzt, daß auf 1 Mol des Acylcyanids überstöchiometrische Mengen des Alkohols bzw. des Alkens, vorzugsweise 1,5–2 Mol kommen. — Eigene Untersuchungen ergaben, daß bei stöchiometrischer Arbeitsweise nach den in DE-OS 2 733 180 und DE-OS 2 733 181 angeführten Beispielen nur unwirtschaftliche Ausbeuten an α-Ketocarbonsäureamiden, wie z. B. Trimethylbrenztraubensäure-N-tert.-butylamid (Ausbeute <45% der Theorie), erhalten werden.

Es wurde nun überraschend gefunden, daß man α-Ketocarbonsäure-N-tert.-butylamide der allgemeinen Formel I

$$R-CO-CO-NH-C(CH_3)_3 \qquad (I)$$

in welcher

R für einen gegebenenfalls substituierten aliphatischen Rest mit bis zu 8 Kohlenstoffatomen, für einen gegebenenfalls substituierten Cycloalkylrest mit 3 bis 6 Kohlenstoffatomen, für einen gegebenenfalls substituierten Phenyl- oder Naphthylrest oder für einen gegebenenfalls substituierten heterocyclischen Rest steht,

mit hohen Ausbeuten und in hoher Reinheit erhält, wenn man Acylcyanide der allgemeinen Formel II

$$R-CO-CN \quad (II)$$

worin R die oben angegebene Bedeutung hat, mit tert.-Butylmethyläther der Formel III

$$(CH_3)_3C-O-CH_3 \quad (III)$$

bei Temperaturen zwischen 0 und 80°C in Gegenwart einer Säure, die den Äther (III) unter den Reaktionsbedingungen zu einem t-Butyl-carboniumion zu aktivieren vermag, und gegebenenfalls in Gegenwart eines Lösungsmittels umsetzt und das Reaktionsgemisch anschließend in an sich bekannter Weise hydrolysiert.

Die erfindungsgemäße Umsetzung wird unter den Bedingungen der vorerwähnten »Ritter-Reaktion« durchgeführt. Dabei ist es besonders überraschend, daß sich — jeweils insbesondere bei etwa stöchiometrischen Ansätzen — mit Hilfe der erfindungsgemäßen Umsetzung wesentlich höhere Ausbeuten erzielen lassen als bei Verwendung von t-Butanol bzw. Isobutylen als t-Butylcarboniumionen-Quelle.

Verwendet man neben t-Butylmethyläther Pivaloylcyanid als Ausgangsstoff und führt die Umsetzung in Gegenwart von konzentrierter Schwefelsäure durch, so kann der Reaktionsablauf durch das folgende Formelschema wiedergegeben werden:

$$(CH_3)_3C-CO-CN + (CH_3)_3C-O-CH_3$$

$$\xrightarrow[H_2O]{H_2SO_4} (CH_3)_3C-CO-CO-NH-C(CH_3)_3$$

Die als Ausgangsstoffe einzusetzenden Acylcyanide sind durch Formel (II) allgemein definiert. In dieser Formel steht R vorzugsweise für geradkettiges oder verzweigtes Alkyl mit 1 bis 4 Kohlenstoffatomen, wobei jeder dieser Alkylreste substituiert sein kann durch Alkoxy mit 1 bis 4 Kohlenstoffatomen, Carbalkoxy mit 1 bis 4 Kohlenstoffatomen in der Alkoxygruppe, Nitro, Nitril und/oder Halogen, wie zum Beispiel Fluor, Chlor, Brom oder Jod; ferner steht R vorzugsweise für gegebenenfalls durch Alkyl, Alkoxy oder Carbalkoxy mit jeweils bis zu 4 Kohlenstoffatomen, Nitro, Nitril und/oder Halogen, wie zum Beispiel Fluor, Chlor und Brom, substituiertes Cycloalkyl mit 5 oder 6 Kohlenstoffatomen im Ringsystem. Weiterhin steht R vorzugsweise für gegebenenfalls durch Alkyl, Alkoxy oder Carbalkoxy mit jeweils bis zu 4 Kohlenstoffatomen, Nitro und/oder Halogen, wie zum Beispiel Fluor,Chlor und Brom, substituiertes Phenyl oder Naphthyl; schließlich steht R vorzugsweise für gegebenenfalls durch Alkyl, Alkoxy oder Carbalkoxy mit jeweils bis zu 4 Kohlenstoffatomen, Nitro, Nitril und/oder Halogen, wie z. B. Fluor, Chlor und Brom, substituierte 5- oder 6gliedrige heterocyclische Reste, die 1 bis 3 Heteroatome wie Sauerstoff, Schwefel und/oder Stickstoff im Ring enthalten können und außerdem mit einem Benzolring annelliert sein können. Als Beispiele für insbesondere in Frage kommende heterocyclische Reste seien genannt: Morpholinyl, Imidazolyl, Pyrazolyl, Pyrrolyl, Isoxazolyl, Piperidinyl, Oxazolyl, 1,2,4-Triazol-1-yl, 1,2,4-Triazol-4-yl, 1,2,3-Triazolyl, 1,2,4-Thiadiazol-2-yl, Benzimidazolyl und Furanyl.

Die Acylcyanide der Formel (II) sind zum Teil bekannt; noch nicht bekannte Acylcyanide können nach bekannten Verfahren hergestellt werden (vgl. Angew. Chem. 68, S. 425–435 [1965]; ferner DE-OS 2 614 240, 2 614 241, 2 614 242, 2 708 182, 2 708 183).

Als im Rahmen dieser Erfindung besonders bevorzugte Acylcyanide seien Pivaloylcyanid und Benzoylcyanid genannt.

Tert.-Butylmethyläther (III) ist großtechnisch verfügbar und im Handel erhältlich.

Die erfindungsgemäße Umsetzung wird in Gegenwart einer Säure, die den Äther (III) unter den Reaktionsbedingungen zu einem t-Butylcarboniumion zu aktivieren vermag, durchgeführt.

Als solche Säuren kommen insbesondere konzentrierte Schwefelsäure, aber auch eine Reihe von weiteren, bei Ritter-Reaktionen gebräuchlichen Säuren in Frage, nämlich bestimmte Sulfonsäuren, Phosphonsäuren sowie Halogenalkancarbonsäuren, von denen im einzelnen die folgenden genannt seien:

a) Sulfonsäuren wie z. B. Methansulfonsäure, Äthansulfonsäure, Propansulfonsäure, Butansulfonsäure, Trifluormethansulfonsäure, Benzolsulfonsäure, Toluolsulfonsäure, Benzylsulfonsäure, 4-(2-Dodecyl)-phenylsulfonsäure, Hexadecylsulfonsäure, Octadecylsulfonsäure, Benzol-1,3-disulfonsäure, Äthan-1,2-disulfonsäure und Butan-1,4-disulfonsäure;
b) aliphatische und aromatische Phosphonsäure wie z. B. Methanphosphonsäure, Äthanphosphonsäure, Phenylphosphonsäure, Benzylphosphonsäure und Äthan-1,2-diphosphonsäure;
c) Halogenalkancarbonsäuren wie z. B. Dichloressigsäure, Trichloressigsäure, Trifluoressigsäure und Perchlorpropionsäure.

Es ist möglich, die erfindungsgemäße Umsetzung in Gegenwart einer oder mehrerer solcher Säuren durchzuführen.

Die Reaktionstemperatur kann in einem größeren Bereich variiert werden. Im allgemeinen arbeitet man, wie oben angegeben, bei Temperaturen zwischen 0 und 80°C, vorzugsweise zwischen 10 und 40°C. Die anschließende Hydrolyse wird zweckmäßig mittels Eiswasser durchgeführt.

Die Reaktion wird im allgemeinen bei Normaldruck durchgeführt.

Die Umsetzung kann in Abwesenheit oder in Gegenwart eines Lösungsmittels bzw. Lösungsvermittlers durchgeführt werden. Als Lösungsvermittler kommen bestimmte organische Lösungsmittel in Frage; besonders geeignet sind Eisessig und Dichlormethan, ferner Dialkyläther, wie Diäthyl- oder Di-isopropyläther, und Diaryläther wie z. B. Diphenyläther. Es kann jedoch auch zweckmäßig sein, überschüssigen t-Butylmethyläther (III) als Lösungsmittel einzusetzen und dann ohne zusätzlichen Lösungsvermittler zu arbeiten.

Bei der Durchführung des erfindungsgemäßen Verfahrens setzt man im allgemeinen — sofern man nicht mit einem beträchtlichen Überschuß an t-Butylmethyläther (III) als Lösungsmittel arbeitet — auf 1 Mol Acylcyanid der Formel (II) 1 bis 1,5 Mol t-Butylmethyläther, vorzugsweise 1 bis 1,2 Mol t-Butylmethyläther ein; besonders bevorzugt ist das stöchiometrische Molverhältnis von 1 Mol t-Butylmethyläther auf 1 Mol eines Acylcyanids der Formel (II). Soll der t-Butylmethyläther zugleich als Lösungsmittel dienen, so kann dieser, wie andere Lösungsmittel auch, praktisch in beliebigem Überschuß eingesetzt werden.

Die für die Durchführung des erfindungsgemäßen Verfahrens erforderlichen Säuren werden in stöchiometrischer oder überstöchiometrischer Menge eingesetzt. Im allgemeinen setzt man auf 1 Mol Acylcyanid der Formel (II) 1 bis 5 Mol, bevorzugt 1,1 bis 1,5 Mol Säure ein.

Zweckmäßigerweise geht man bei der Durchführung des Verfahrens so vor, daß man die Säure bzw. ein Gemisch aus Lösungsmittel und Säure vorlegt und ein Gemisch der beiden übrigen Komponenten, Acylcyanid und t-Butylmethyläther, gegebenenfalls in Lösungsmittel, zugibt.

Die Reaktionszeiten betragen im allgemeinen 1—10 Stunden. Zur Hydrolyse wird das Reaktionsgemisch anschließend am zweckmäßigsten auf Eis gegossen. Die gebildeten α-Ketocarbonsäureamide können durch Filtration oder durch Extraktion isoliert werden.

Hierzu geeignete Extraktionsmittel sind mit Wasser nicht beliebig mischbare Lösungsmittel, beispielsweise Äther wie Diethyläther oder Diisopropyläther, Ester wie z. B. Essigsäureethylester, Ketone wie z. B. Methylisobutylketon, Halogenkohlenwasserstoffe wie z. B. Dichlormethan, Chlorbenzol oder Dichlorbenzol, ferner Aromaten wie z. B. Benzol, Toluol, o-Xylol, Ethylbenzol, Cumol oder Nitrobenzol. Vorzugsweise verwendet man Dichlormethan. Weiterhin ist es möglich, überschüssigen, als Lösungsmittel eingesetzten und nicht in die Reaktion eingetretenen tert.-Butylmethyläther (III) bei der Aufarbeitung zugleich als Extraktionsmittel zu verwenden; dieser kann nach einer Destillation wiederverwendet werden.

Die erfindungsgemäß herstellbaren α-Ketocarbonsäureamide der Formel (I) sind teilweise bekannt; sie können z. B. als Zwischenprodukte zur Synthese von herbiziden Wirkstoffen verwendet werden. So erhält man beispielsweise aus dem Trimethylbrenztraubensäure-N-tert.-butylamid (Ia) nach folgendem Reaktionsschema die herbizid besonders wirksame Verbindung 4-Amino-6-tert.-butyl-3-methylthio-1,2,4-triazin-5(4H)-on (VI) (vgl. DE-PS 1 795 784):

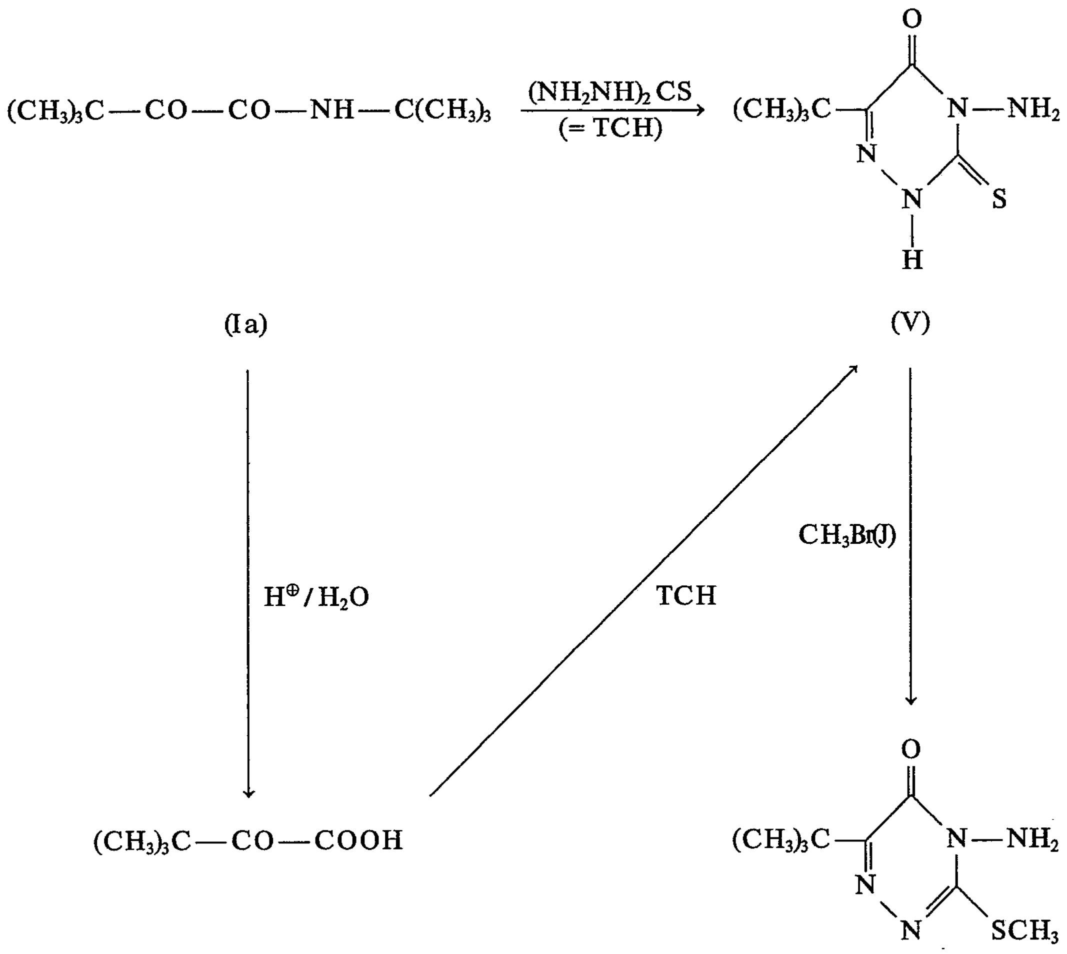


(Ia) (V) (IV) (VI)

Trimethylbrenztraubensäure-N-tert.-butylamid (Ia) kann in bekannter Weise entweder direkt oder nach vorheriger Verseifung zur freien α-Ketosäure (IV) in halogenwasserstoffsaurer, wäßriger Lösung mit 1 bis 1,5 Mol Thiocarbohydrazid, $NH_2-NH-CS-NH-NH_2$ (=TCH), bei Temperaturen zwischen 20 und 100°C zu 4-Amino-6-tert.-butyl-3-mercapto-1,2,4-triazin-5(4H)-on (V) kondensiert werden, welches sich mittels Methylhalogenid, z. B. Methyljodid oder Methylbromid, in alkalischer Lösung zu (VI) methylieren läßt (vgl. Chem. Ber. 97, S. 2173–8 [1964]; DE-OS 2 165 554; DE-OS 2 460 889; DE-OS 2 648 300; DE-OS 2 733 180).

Die nachfolgenden Herstellungsbeispiele sollen zur näheren Erläuterung des erfindungsgemäßen Verfahrens dienen.

## Herstellungsbeispiele

### Beispiel 1

$$(CH_3)_3C-CO-CO-NH-C(CH_3)_3$$

Dieses Beispiel veranschaulicht eine bevorzugte Arbeitsweise gemäß der Erfindung:

In eine vorgelegte Reaktionsmischung, bestehend aus 37,5 g konzentrierter Schwefelsäure und 52,5 g Eisessig werden 27,8 g (0,25 Mol) Pivaloylcyanid, gelöst in 22 g (0,25 Mol) t-Butylmethyläther, innerhalb von 30 Minuten bei 20–30°C eingetragen. Nach 2stündigem Nachrühren bei Raumtemperatur wird die Reaktionsmischung auf 125 g Eis gegossen und gut durchgerührt. Das ausfallende Produkt wird abgesaugt, mit Wasser gewaschen und getrocknet. Man erhält 40,8 g (87,5% der Theorie) analysenreines Trimethylbrenztraubensäure-N-tert.-butylamid vom Schmelzpunkt 65°C, mit einem Gehalt von >99% (gaschromatographisch bestimmt).

Vergleichsbeispiele

a) Das folgende Vergleichsbeispiel veranschaulicht eine dem Stand der Technik entsprechende Arbeitsweise, wie sie z. B. in Beispiel 1 der DE-OS 2 733 180 beschrieben ist. Jedoch wird die Carboniumionenquelle t-Butanol — zwecks Vergleich mit dem zuvor beschriebenen erfindungsgemäßen Beispiel 1 — in stöchiometrischer Menge mit Pivaloylcyanid umgesetzt.
27,8 g (0,25 Mol) Pivaloylcyanid werden zu einer Mischung aus 18,5 g (0,25 Mol) t-Butanol mit 12,5 ml Methylenchlorid gegeben. Dann tropft man unter Rühren bei 0–5° C 37,5 g 98%ige Schwefelsäure zu, anschließend erwärmt man auf 20° C. Nach 4stündigem Nachrühren wird das Reaktionsgemisch auf 100 g Eis gegossen und weitere 30 Minuten gerührt. Man verdünnt anschließend mit 75 ml Methylenchlorid, trennt die organische Phase ab und dampft die Methylenchloridlösung ein. Es bleiben 47,0 g eines gelben öligen Produktes zurück, dessen Gehalt an gewünschtem Trimethylbrenztraubensäure-N-tert.-butylamid 33% beträgt (gaschromatographisch bestimmt), so daß eine Ausbeute von 33,5% der Theorie resultiert.

b) Das folgende Vergleichsbeispiel zeigt eine dem Stand der Technik entsprechende Arbeitsweise, wie sie z. B. in Beispiel 3 der DE-OS 2 733 181 beschrieben ist. Wie im Vergleichsbeispiel a) wird die hier eingesetzte Carboniumionquelle Isobutylen in stöchiometrischer Menge mit Pivaloylcyanid umgesetzt.
In einer gegen Feuchtigkeit geschützten Rührapparatur werden, wie in Beispiel 3 der DE-OS 2 733 181 beschrieben, 27,8 g (0,25 Mol) Pivaloylcyanid mit 14 g (0,25 Mol) Isobutylen umgesetzt. Man erhält 20,4 g rohes Trimethylbrenztraubensäure-N-tert.-butylamid mit einem Gehalt von 93,8% (gaschromatographisch bestimmt), was einer Ausbeute von 41,3% der Theorie entspricht.

c) Es wird wie im Vergleichsbeispiel b) verfahren, jedoch wird die Aufarbeitung der stöchiometrischen Umsetzung von Pivaloylcyanid mit Isobutylen wie im Beispiel 1b) der DE-OS 2 733 180 beschrieben durchgeführt.
Man erhält 22,0 g rohres Trimethylbrenztraubensäure-N-tert.-butylamid mit einem Gehalt von 93,5% (gaschromatographisch bestimmt), was einer Ausbeute von 44,4% der Theorie entspricht.

Beispiel 2

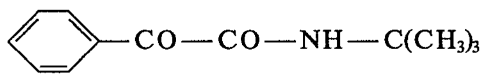

Dieses Beispiel veranschaulicht eine bevorzugte Arbeitsweise gemäß der Erfindung.

In eine vorgelegte Reaktionsmischung, bestehend aus 37,5 g konzentrierter Schwefelsäure und 52,5 g Eisessig werden 32,8 g (0,25 Mol) Benzoylcyanid, gelöst in 22 g (0,25 Mol) t-Butylmethyläther, innerhalb von 30 Minuten bei 20–30°C eingetragen. Nach 2stündigem Nachrühren bei Raumtemperatur wird die Reaktionsmischung auf 125 g Eis gegossen und gut durchgerührt. Das ausfallende Produkt wird abgesaugt, mit Wasser gewaschen und getrocknet. Man erhält 45,6 g (88,9% der Theorie) analysenreines Phenylglyoxylsäure-N-tert.-butylamid vom Schmelzpunkt 78° C, mit einem Gehalt von $>$99% (gaschromatographisch bestimmt).

Vergleichsbeispiele

a) Das folgende Vergleichsbeispiel zeigt eine dem Stand der Technik entsprechende Arbeitsweise, wie sie z. B. in Beispiel 1 der DE-OS 2 733 181 beschrieben ist, wobei jedoch t-Butanol mit der stöchiometrischen Menge an Benzoylcyanid umgesetzt wird:
Man erhält 37,1 g Phenylglyoxylsäure-N-tert.-butylamid mit einem (gaschromatographisch bestimmten) Gehalt von 91,6%, was einer Ausbeute von 66,2% der Theorie entspricht.

b) Es wird wie in Beispiel 3 der DE-OS 2 733 181 beschrieben verfahren, wobei jedoch Isobutylen mit der stöchiometrischen Menge an Benzoylcyanid umgesetzt wird.
Man erhält 26,9 g Phenylglyoxylsäure-N-tert.-butylamid mit einem (gaschromatographisch bestimmten) Gehalt von 90,8%, was einer Ausbeute von 47,6% der Theorie entspricht.

**Patentansprüche**

1. Verfahren zur Herstellung von $\alpha$-Ketocarbonsäure-N-tert.-butylamiden der allgemeinen Formel I

$$R-CO-CO-NH-C(CH_3)_3 \qquad (I)$$

in welcher
R für einen gegebenenfalls substituierten aliphatischen Rest mit bis zu 8 Kohlenstoffatomen, für einen

gegebenenfalls substituierten Cycloalkylrest mit 3 bis 6 Kohlenstoffatomen, für einen gegebenenfalls substituierten Phenyl- oder Naphthylrest oder für einen gegebenenfalls substituierten heterocyclischen Rest steht,
dadurch gekennzeichnet, daß man Acylcyanide der allgemeinen Formel II

$$R-CO-CN \qquad (II)$$

in welcher R die oben angegebene Bedeutung hat,
mit tert.-Butylmethyläther der Formel III

$$(CH_3)_3C-O-CH_3 \qquad (III)$$

bei Temperaturen zwischen 0 und 80°C in Gegenwart einer Säure, die den Äther (III) unter den Reaktionsbedingungen zu einem tert.-Butylcarboniumion zu aktivieren vermag, und gegebenenfalls in Gegenwart eines Lösungsmittels umsetzt und das Reaktionsgemisch in an sich bekannter Weise hydrolysiert.

2. Verfahren nach Anspruch 1, dadurch gekennzeichnet, daß man die Umsetzung bei Temperaturen zwischen 10 und 40° C durchführt.

3. Verfahren nach Anspruch 1, dadurch gekennzeichnet, daß Acylcyanid (II) und tert.-Butylmethyläther (III) im Molverhältnis 1 : 1—1,5 umgesetzt werden.

4. Verfahren nach Anspruch 4, dadurch gekennzeichnet, daß Acylcyanid (II) und tert.-Butylmethyläther (III) im (stöchiometrischen) Molverhältnis 1 : 1 umgesetzt werden.

5. Verfahren nach Anspruch 1, dadurch gekennzeichnet, daß als Acylcyanid (II) Pivaloylcyanid, $(CH_3)_3C-CO-CN$, eingesetzt wird.

6. Verfahren nach Anspruch 1, dadurch gekennzeichnet, daß als Acylcyanid (II) Benzoylcyanid, $C_6H_5-CO-CN$, eingesetzt wird.

7. Verfahren nach Anspruch 1, dadurch gekennzeichnet, daß als Lösungsmittel Eisessig oder Dichlormethan eingesetzt wird.

8. Verfahren nach Anspruch 1, dadurch gekennzeichnet, daß man auf 1 Mol Acylcyanid (II) 1—5 Mol, vorzugsweise 1,1—1,5 Mol Säure einsetzt.

9. Verfahren nach Anspruch 1, dadurch gekennzeichnet, daß man als Säure konzentrierte Schwefelsäure einsetzt.

10. Verfahren nach Anspruch 1, dadurch gekennzeichnet, daß man bei der Durchführung des Verfahrens die Säure bzw. ein Gemisch aus Lösungsmittel und Säure vorlegt und ein Gemisch von Acylcyanid (II) und tert.-Butylmethyläther (III), gegebenenfalls in Lösungsmittel, zugibt.

**Claims**

1. Process for the preparation of α-ketocarboxylic acid N-tert.-butylamides of the general formula I

$$R-CO-CO-NH-C(CH_3)_3 \qquad (I)$$

in which
R represents an optionally substituted aliphatic radical with up to 8 carbon atoms, an optionally substituted cycloalkyl radical with 3 to 6 carbon atoms, an optionally substituted phenyl or naphthyl radical or an optionally substituted heterocyclic radical,
characterised in that acyl cyanides of the general formula II

$$R-CO-CN \qquad (II)$$

in which
R has the meaning indicated above,
are reacted with tert.-butyl methyl ether of the formula III

$$(CH_3)_3C-O-CH_3 \qquad (III)$$

at temperatures between 0 and 80° C in the presence of an acid which is capable of activating the ether (III) under the reaction conditions to give a tert.-butyl-carbonium ion, and if appropriate in the presence of a solvent, and the reaction mixture is then hydrolysed in a manner which is in itself known.

2. Process according to Claim 1, characterised in that the reaction is carried out at temperatures between 10 and 40° C.

3. Process according to Claim 1, characterised in that the acyl cyanide (II) and tert.-butyl methyl ether (III) are reacted in a molar ration of 1 : 1—1.5.

4. Process according to Claim 4, characterised in that the acyl cyanide (II) and thert.-butyl methyl

ether (III) are reacted in a (stoichiometric) molar ratio of 1 : 1.

5. Process according to Claim 1, characterised in that pivaloyl cyanide, that is to say $(CH_3)_3C-CO-CN$, is employed as the acyl cyanide (II).

6. Process according to Claim 1, characterised in that benzoyl cyanide, that is to say $C_6H_5-CO-CN$, is employed as the acyl cyanide (II).

7. Process according to Claim 1, characterised in that glacial acetic acid or methylene chloride is employed as the solvent.

8. Process according to Claim 1, characterised in that 1 – 5 mols, preferably 1.1 – 1.5 mols, of acid are employed per 1 mol of acyl cyanide (II).

9. Process according to Claim 1, characterised in that concentrated sulphuric acid is employed as the acid.

10. Process according to Claim 1, characterised in that, in carrying out the process, the acid or a mixture of the solvent and the acid is initially introduced and a mixture of the acyl cyanide (II) and tert.-butyl methyl ether (III), if appropriate in a solvent, is added.

**Revendications**

1. Procédé de production de N-tertio-butylamides d'acides $\alpha$-cétocarboxyliques de formule générale I

$$R-CO-CO-NH-C(CH_3)_3 \qquad (I)$$

dans laquelle

R représente un reste aliphatique éventuellement substitué ayant jusqu'à 8 atomes de carbone, un reste cycloalkyle éventuellement substitué ayant 3 à 6 atomes de carbone, un reste phényle ou naphtyle éventuellement substitué ou un reste hétérocyclique éventuellement substitué,

caractérisé en ce qu'on fait réagir des cyanures d'acyle de formule générale II

$$R-CO-CN \qquad (II)$$

dans laquelle

R a la définition indiquée ci-dessus,

avec l'éther de tertio-butyle et de méthyle de formule III

$$(CH_3)_3C-O-CH_3 \qquad (III)$$

à des températures comprises entre 0 et 80° C en présence d'un acide qui peut activer l'éther (III) dans les conditions réactionnelles en formant un ion tertio-butylcarbonium et, le cas échéant, en présence d'un solvant, et on hydrolyse d'une manière connue le mélange réactionnel.

2. Procédé suivant la revendication 1, caractérisé en ce qu'on conduit la réaction à des températures comprises entre 10 et 40° C.

3. Procédé suivant la revendication 1, caractérisé en ce qu'on fait réagir le cyanure d'acyle (II) et l'éther de tertio-butyle et de méthyle (III) dans un rapport molaire de 1 : 1 – 1,5.

4. Procédé suivant la revendication 4, caractérisé en ce qu'on fait réagir le cyanure d'acyle (II) et l'éther de tertio-butyle et de méthyle (III) dans un rapport utilise (stoechiométrique) de 1 : 1.

5. Procédé suivant la revendication 1, caractérisé en ce qu'on utilise comme cyanure d'acyle (II) le cyanure de pivaloyle $(CH_3)_3C-CO-CN$.

6. Procédé suivant la revendication 1, caractérisé en ce qu'on utilise comme cyanure d'acyle (II) le cyanure de benzoyle $C_6H_5-CO-CN$.

7. Procédé suivant la revendication 1, caractérisé en ce qu'on utilise comme solvant l'acide acétique cristallisable ou le dichlorométhane.

8. Procédé suivant la revendication 1, caractérisé en ce qu'on utilise par mole de cyanure d'acyle (II), 1 à 5 moles, de préférence 1,1 à 1,5 mole d'acide.

9. Procédé suivant la revendication 1, caractérisé en ce qu'on utilise comme acide de l'acide sulfurique concentré.

10. Procédé suivant la revendication 1, caractérisé en ce que, dans la mise en oeuvre du procédé, on introduit tout d'abord l'acide ou un mélange du solvant et de l'acide et on ajoute un mélange du cyanure d'acyle (II) et de l'éther de tertio-butyle et de méthyle (III), éventuellement dans un solvant.